# EUROPÄISCHE PATENTANMELDUNG

(11) **EP 0 982 588 A1**
(43) Veröffentlichungstag der Anmeldung: **01.03.2000**
(21) Anmeldenummer: 98115965.0
(22) Anmeldetag: 25.08.1998
(51) Int. Cl.: G01N 33/00, G01N 29/02, G01N 27/00, G08B 17/10

(54) **Brandmelder mit einem Sensor zur Rauchdetektion und Verfahren zur Herstellung eines Sensors mit molekularer Prägung zur Rauchdetektion**

(71) Anmelder: Siemens Building Technologies AG, 8708 Männedorf (CH)
(72) Erfinder: Forster, Martin, Dr., 8645 Jona (CH)
(74) Vertreter: Dittrich, Horst Dr.

(57) **Zusammenfassung**

Der Brandmelder enthält einen Sensor (6, 7) zur Rauchdetektion und eine Auswerteschaltung (5) für die Signale des Sensors. Der Sensor besteht aus einem eine Sensorschicht mit molekularer Prägung für den Rauchnachweis aufweisenden Rauchsensor (6) und aus einem Referenzsensor (7) mit einer vom Rauchsensor abweichenden Rauchselektivität. Der Rauch- und der Referenzsensor (6 bzw. 7) sind je durch einen Massensensor gebildet.

Zur Herstellung des Rauchsensors (6) werden in ein Gemisch von Matrixmolekülen Rauchgasmoleküle eingebracht und die dadurch entstehende Mischung wird auf das Substrat eines Massensensors aufgebracht und gehärtet. Anschliessend werden die Rauchgasmoleküle aus der Matrix entfernt, wobei in dieser Poren zurückbleiben, in welche die Rauchgasmoleküle exakt hineinpassen.

## Beschreibung

Die vorliegende Erfindung betrifft einen Brandmelder mit einem Sensor zur Rauchdetektion und mit einer Auswerteschaltung für die Signale des Sensors.

Heute übliche Sensoren zur Rauchdetektion beruhen in erster Linie auf der Streuung der von einer Lichtquelle ausgesandten Strahlung an Rauchpartikeln (sogenannte Streulichtmelder, siehe beispielsweise EP-A-0 821 331) oder auf der Abschwächung eines Lichtstrahls durch Rauch (sogenannte Extinktionsmelder, siehe beispielsweise EP-A-0 813 178) oder auf der Änderung der optischen Transmission oder der spektralen Absorption, oder auf der Lichtstreuung an Aerosolen oder auf der Oberflächenionisation (siehe dazu EP-A-0 787 985). Dabei ist all diesen verschiedenen Nachweisverfahren gemeinsam, dass sie in der Regel für bestimmte Arten von Rauch besser und für andere Arten von Rauch weniger gut geeignet sind.

Durch die Erfindung soll nun ein Sensor zur Rauchdetektion angegeben werden, der auf alle vorkommenden Arten von Rauch und Rauchgasen etwa gleich empfindlich ist und der möglichst geringe Rauchkonzentrationen nachweisen kann. Ausserdem soll es möglich sein, den Sensor an spezielle Rauchtypen anpassen und dadurch durch die gleichzeitige Verwendung eines breitbandigen und eines schmalbandigen Sensors eine extreme Fehlalarmsicherheit erreichen zu können.

Die gestellte Aufgabe wird erfindungsgemäss dadurch gelöst, dass der Sensor aus einem eine Sensorschicht mit molekularer Prägung für den Rauchnachweis aufweisenden Rauchsensor und aus einem Referenzsensor mit einer vom Rauchsensor abweichenden Rauchselektivität besteht.

Unter molekularer Prägung versteht man im wesentlichen das Aufbringen des nachzuweisenden Moleküls zusammen mit einem Monomer in dünner Schicht auf ein geeignetes Substrat, die anschliessende Polymerisation des Monomers und Entfernung der nachzuweisenden Moleküle aus dem Polymer durch Auswaschen oder Verdampfen, wobei im Polymer Hohlräume und Diffusionspfade entstehen, die exakt auf die nachzuweisenden Moleküle passen. Wenn diese Polymerschicht in eine Umgebung gebracht wird, in der die nachzuweisende Substanz vermutet wird, dann lagern sich die nachzuweisenden Moleküle selektiv in die Polymerschicht ein, was durch geeignete Methoden nachgewiesen werden kann. Der Referenzsensor, in dessen Polymerschicht keine nachzuweisenden Moleküle eingelagert sind, dient dazu, eventuelle durch Ablagerung von Staub und anderen Verunreinigungen auf dem Sensor verursachte Störungseinflüsse zu eliminieren Eine erste bevorzugte Ausführungsform des erfindungsgemässen Brandmelders ist dadurch gekennzeichnet, dass der Rauch- und der Referenzsensor je durch einen Massensensor gebildet sind.

Massensoren zeigen die Anwesenheit von Substanzen an, indem sie die nachzuweisenden Substanzen adsorbieren oder absorbieren und dadurch an Masse zunehmen. Aus der Grösse des Massenzuwachses kann dann auf die Menge der nachzuweisenden Substanz geschlossen werden. Beispiele solcher Massensensoren sind Schwingquarze in Dickenschwingung, Surface Acoustic Wave (SAW) Resonatoren und Schwingbalken, je mit aufgebrachter Adsorptions-/Absorptionsschicht. Allen diesen Sensoren ist eine sehr hohe Empfindlichkeit und ein sehr geringer Leistungsverbrauch gemeinsam.

Die schon erwähnte Ablagerung von Verunreinigungen auf dem Sensor führt ebenfalls zu einem Massenzuwachs, der sich dem durch die Ad- oder Absorption der nachzuweisenden Substanzen verursachten Massenzuwachs überlagert und dadurch das Messergebnis verfälscht. Mit Hilfe des Referenzsensors kann der durch diese Ablagerungen erzeugte Massenzuwachs bestimmt und vom Signal des Rauchsensors subtrahiert werden.

Die Erfindung betrifft weiter ein Verfahren zur Herstellung eines Sensors mit molekularer Prägung zur Rauchdetektion. Dieses Verfahren ist dadurch gekennzeichnet, dass in ein Gemisch von Matrixmolekülen Rauchgasmoleküle eingebracht werden und die dadurch entstehende Mischung auf ein Substrat aufgebracht und gehärtet wird, und dass anschliessend die Rauchgasmoleküle aus der Matrix entfernt werden, wobei in dieser Poren zurückbleiben, in welche die Rauchgasmoleküle exakt hineinpassen.

Eine erste bevorzugte Ausführungsform des erfindungsgemässen Verfahrens ist dadurch gekenzeichnet, dass die Härtung der genannten Mischung durch Polymerisierung erfolgt.

Der als Referenzsensor wirkende unselektive Massensensor wird ähnlich wie der selektive hergestellt und unterscheidet sich von diesem im wesentlichen dadurch, dass auf das Substrat des unselektiven Massensensors nur das aus den Matrixmolekülen bestehende Monomer, ohne Beimischung der Rauchgasmoleküle, aufgebracht wird. Die Polymerschicht des unselektiven Massensensors enthält also keine Diffusionspfade und keine Hohlräume für die selektive Aufnahme von Rauchgasmolekülen, so dass dieser Referenzsensor nur die Verstaubung und die Verschmutzung misst.

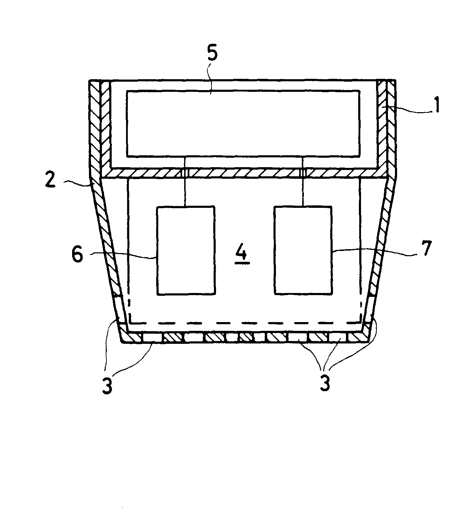

Im folgenden wird die Erfindung anhand eines Ausführungsbeispiels näher erläutert, wobei die einzige Zeichnung eine schematische Darstellung der Messkammer eines erfindungsgemässen Brandmelders mit einem Massensensor zeigt.

Der erfindungsgemässe Brandmelder besteht in bekannter Weise aus einem Meldereinsatz 1, der in einem vorzugsweise an der Decke des zu überwachenden Raumes montierten Sockel (nicht dargestellt) befestigbar ist, und aus einer über den Meldereinsatz 1 gestülpten Melderhaube 2 die am Boden und an der Seitenwand mit Raucheintrinsöffnungen 3 versehen ist. Der Meldereinsatz 1 umfasst im wesentlichen einen schachtelartigen Basiskörper, an dessen gegen den zu überwachenden Raum gerichteter unterer Seite eine Messkammer 4 und an dessen dem Sockel zugewandter oberer Seite eine Auswerteelektronik 5 angeordnet ist.

Die Messkammer 4, die in bekannter Weise gegen Staubpartikel und Insekten möglichst gut abgedichtet und für Rauch möglichst frei zugänglich ist, enthält einen selektiven und einen unselektiven Massensensor, von denen der erstere als Rauchsensor 6 und der letztere als Referenzsensor 7 dient. Die beiden Sensoren 6 und 7 sind an die Auswerteelektronik 5 angeschlossen und so in der Messkammer 4 angeordnet, dass sie für in die Messkammer eindringenden Rauch identische Anströmbedingungen aufweisen. Der Rauchsensor 6 und der Referenzsensor 7 sind beispielsweise je durch einen Schwingquarz in Dickenschwingung, einen Surface Acoustic Wave Resonator oder einen Schwingbalken, je mit aufgebrachter Adsorptions-/Absorptionsschicht, gebildet.

Diesen verschiedenen Sensortypen ist folgendes gemeinsam:
■ Die Empfindlichkeit ist sehr hoch und kann bis zu ppb reichen, und der Leistungsverbrauch ist sehr gering.
■ Das Messprinzip als solches ist unselektiv. Selektivität erreicht man entweder durch eine entsprechende Selektivität der aufgebrachten Adsorptions-/Absorptionsschicht oder dadurch, dass nur die Moleküle einer selektierten Substanz bis zum Massensensor durchgelassen werden.
■ Wenn in der Adsorptions-/Absorptionsschicht die Moleküle absorbiert, also fix eingelagert werden, erhält man das zeitliche Integral über die in der Umgebung des Massensensors vorhandene Konzentration an nachzuweisender Substanz.
■ Wenn in der Adsorptions-/Absorptionsschicht die Moleküle adsorbiert und später wieder desorbiert, also nur so lange eingelagert werden, wie die nachzuweisende Substanz in der Nähe des Massesensors vorhanden ist, erhält man die in der Umgebung des Massensensors vorhandene Konzentration an nachzuweisender Substanz. ■ Die zugehörige Elektronik ist einfach und billig und benötigt nur wenig Leistung.

Das Prinzip der molekularen Prägung, durch welches der Massensensor die gewünschte Selektivität erhält, wird beispielsweise zum selektiven Nachweis von Substanzen durch ihre Fluoreszenz in optochemischen Sensoren benützt [F.L. Dickert, S. Thierer, Adv. Mater. 8 (1996) 987] oder zur Reinigung oder Analyse von Lösungen, die makromolekulare Biomoleküle, wie Proteine, Enzyme, usw. enthalten (EP-A-0 602 154).

Bei der molekularen Prägung zum Rauchnachweis werden aus den Rauchgasen der verschiedenen Feuer die Rauchgasmoleküle abgetrennt und mit einem geeigneten Monomer, z.B. Styrol, vermischt. Dann wird das Gemisch auf das Substrat des Massensensors gebracht und das Monomer wird polymerisiert (z.B. durch Wärme, Licht oder eine sonstige geeignete Strahlung), wobei durch geeignete Masken die erzielte Polymerschicht strukturiert erhalten wird. Schliesslich werden die Rauchgasmoleküle entfernt (z.B. durch Wärme, Vakuum oder Auswaschen mit einem Lösungsmittel), wodurch die Polymerschicht Hohlräume und Diffusionspfade erhält, die exakt auf die Rauchgasmoleküle passen, so dass sich diese sclektiv in die Polymerschicht einlagern können.

Die durch die selektive Einlagerung bewirkte Massenänderung der Polymerschicht wird anhand der entsprechenden Änderung der Resonanzfrequenz des Rauchsensors 6 gemessen, was mit einem einfachen Oszillator erfolgt. Diese Frequenzänderung ist dann das in der Auswerteelektronik 5 zu verarbeitende Signal des Rauchsensors 6.

Nachfolgend sollen einige Beispiele für die Herstellung und Anwendung von molekulargeprägten Rauchsensoren gegeben werden:

### Beispiel 1:

Der Rauch eines Holzfeuers wurde mittels Unterdruck angesaugt und durch eine Wasserflasche von 3 Litern Inhalt, gefüllt mit 1,4-Dioxan geleitet. Alle bei Raumtemperatur flüssigen/festen Substanzen haben sich im Dioxan gelöst/suspendiert und konnten so abgeschieden werden. Dann wurde die 1,4-Dioxanlösung filtriert und sorgfältig eingedampft, um von den leichtflüchtigen Komponenten möglichst wenig zu verlieren. Zurück blieb ein dickflüssiges Gemisch von Rauchbestandteilen, das in einer Konzentration von ca. 1 bis 5% in Styrol und wenig N,N'-Methylen-bis-acrylamid als Vernetzer gelöst wurde. Diese Lösung wurde auf die Oberfläche eines SAW (Surface Acoustiv Wave)Resonators von Siemens Matshushita vom Typ R2632 aufgebracht und es wurde durch Spinnen des Resonators eine Schicht von Monomeren und Rauchbestandteilen mit einer Dicke von ca. 1 Mikron erzeugt, was den Rauchsensor 6 ergab.

Analog wurde ein gleicher SAW-Resonator mit einer Schicht aus reinem Styrol und wenig N,N'-Methylen-bis-acrylamid hergestellt, was den Referenzsensor 7 ergab. Anschliessend wurden die Monomerschichten im aktiven Teil der SAW-Resonatoren mit UV-Licht bestrahlt, was dort das Styrol anpolymerisierte. Dann wurde unpolymerisiertes Styrol kurz mit Alkohol abgewaschen und die beiden SAW-Resonatoren wurden während mehrerer Stunden auf ca. 80°C erwärmt, wobei einerseits das Styrol fertig polymerisiert und vernetzt wurde und andererseits die Rauchmoleküle im Rauchsensor komplett verdampften und entsprechende Poren in der Polymerschicht zurückliessen. Die beiden SAW-Resonatoren wurden elektronisch so betrieben, dass die Differenz ihrer beiden Resonanzfrequenzen gemessen wurde. Der Betrag dieser Differenzfrequenz ist bei Anwesenheit von Rauch jeweils stark angestiegen.

### Beispiel 2:

Der Rauch eines Luntenbrandes wurde mittels Unterdruck angesaugt und durch eine Kühlfalle von 12 Litern Inhalt geleitet, welche von aussen mit flüssigem Stickstoff auf einer Temperatur von ca. 80°K gehalten wurde, so dass alle bei dieser Temperatur festen Substanzen in der Kühlfalle abgeschieden wurden. Dann wurde die Kühlfalle vorsichtig auf ca. 10°C erwärmt und unter Stickstoff mit Styrol abgespült. Das Styrol wurde filtriert und enthielt nun alle Rauchbestandteile, die sich bei dieser Temperatur im Styrol lösen konnten.

Diese Lösung der Rauchbestandteile in Styrol wurde auf die Oberfläche eines SAW-Resonators von Siemens Matshushita vom Typ R2632 aufgebracht und es wurde durch Spinnen des Resonators eine Schicht von Styrol und Rauchmolekülen mit einer Dicke von unterhalb 1 Mikron erzeugt. Das ergab den Rauchsensor 6; der Referenzsonsor 7 wurde durch einen gleichen SAW-Resonator mit einer Schicht aus reinem Styrol hergestellt. Anschliessend wurden die Stylolschichten im aktiven Teil der SAW-Resonatoren mit UV-Licht bestrahlt, wodurch dort das Stylol anpolymerisiert wurde. Die weitere Verarbeitung erfolgt wie bei Beispiel 1 und ergab einen Sensor, der den Rauch eines Luntenbrandes empfindlich nachweisen konnte.

### Beispiel 3:

50 Zigaretten wurden in eine Ansaughalterung gesteckt und angezündet. Der Rauch dieser Zigaretten wurde mittels Unterdruck angesaugt und durch eine Kühlfalle von 12 Litern Inhalt geleitet, welche von aussen mit flüssigem Stickstoff auf einer Temperatur von ca. 80°K gehalten wurde, so dass alle bei dieser Temperatur festen Substanzen in der Kühlfalle abgeschieden wurden. Dann wurde die Kühlfalle vorsichtig auf ca. 20°C erwärmt und mit einer Mischung aus Methacrylsäure und Vinylpiridin abgespült. Die Lösung wurde filtriert und enthielt nun alle Rauchbestandteile, die sich bei dieser Temperatur in ihr lösen konnten. Diese Lösung der Rauchbestandteile in der Monomerenmischung wurde mit N,N'-Methylen-bis-acrylamid als Vernetzer und Dibenzoylperoxid als Radikalkatalysator versetzt und auf die Oberfläche eines Cantilevers von ca. 400 mal 100 Mikron Fläche aufgebracht, und durch Spinnen der Einrichtung wurde eine Schicht von Lösung und Rauchbestandteilen mit ciner Dicke von unterhalb einem Mikron erzeugt.

Dann wurde die Einrichtung auf ca. 100°C erwärmt, wobei die Mischung auspolymerisierte und vernetzte und gleichzeitig die Rauchbestandteile abdampften und entsprechende Poren in der Schicht hinterliessen. Zur weiteren Reinigung wurde die Cantilever-Einrichtung noch in deionisiertem Wasser gründlich gewaschen. Das ergab den Rauchsensor, der durch die verwendeten Monomere die Carbonsäuren und Amide im Zigarettenrauch selektiv in den Poren der Polymerschicht adsorbieren kann. Analog wurde eine gleiche Cantilever-Einrichtung mit einer Schicht aus reinen Monomeren ohne Rauchbestandteile als Referenzsensor hergestellt.

Die beiden Cantilever-Einrichtungen wurden so betrieben, dass die Differenzfrequenz der beiden Resonanzfrequenzen gemessen wurden. Bei Anwesenheit von Zigarettenrauch hat sich die Resonanzfrequenz des Rauchsensors 6 wesentlich stärker geändert als die Resonanzfrequenz des Referenzsensors 7, so dass der Betrag der Differenzfrequenz der beiden Cantilever-Einrichtungen mit zunehmendem Zigarettenrauch anstieg. Da bei diesem Sensor die Bestandteile des Zigarettenrauchs in der Sensorschicht mit schwachen chemischen Bindungen festgehalten werden, wurden die Cantilever-Einrichtungen periodisch auf ca. 60°C aufgeheizt, um die Sensorschicht wieder von Rauchgasbestandteilen zu reinigen.

Selbstverständlich können anstatt der in den obigen Beispielen beschriebenen Monomere /Polymere, Vernetzer und Katalysatoren auch beliebige andere, dem Fachmann bekannte Systeme eingesetzt werden. Ebenfalls brauchen auch die in die Polymerschichten eingebrachten Rauchgasanteile nicht direkt vom Rauch eines Feuers oder Brandes zu stammen, sondern können auch aus einer synthetisch hergestellten Mischung solcher Substanzen bestehen. Solche synthetische Substanzgemische werden selbstverständlich nicht zuerst in Kühlfallen oder Waschflaschen auskondensiert bzw. ausgewaschen, sondern direkt als Substanzgemisch für die Herstellung der molekular geprägten Sensorschichten eingesetzt. Das Verfahren ist auch nicht auf die Verwendung von SAW-Resonatoren oder Cantilvern beschränkt; es ist auch der Einsatz von Quarz-Dickenschwingern (TSM-Resonatoren) oder sonstigen Massensensoren möglich.

Ebenso können ganze Arrays aus z.B. Cantilevcrn mit molekular geprägten Sensorschichten der beschriebenen Art versehen und dadurch verschiedene Cantilver eines Arrays auf verschiedene Rauchtypen empfindlich gemacht werden. So könnten beispielsweise einige Cantilever den Rauch von Holzbränden, andere den Rauch von Luntenbränden und wieder andere Zigarettenrauch nachweisen. Ebenso ist es auch möglich, zum Nachweis der Rauchgasmoleküle anstatt der massenabhängigen Frequenzverschiebungen andere, beispielsweise optische, Ausleseprinzipien zu verwenden.

Allen diesen Varianten ist aber folgendes gemeinsam:
■ Es werden molekular geprägte Sensorschichten verwendet, wobei als Prägemoleküle diejenigen Moleküle verwendet werden, die im Rauch eines beliebigen Feuers auftreten.
■ Die Selektivität der Sensoren auf Rauch wird dadurch erzeugt, dass in ein Gemisch von Matrixmolekülen Rauchbestandteile eingebracht werden und die Mischung aus Matrixmolekülen und Rauchbestandteilen durch Polymerisierung oder Trocknung/ Härtung der Matrixmoleküle gehärtet wird. Dann werden durch Erwärmung und/oder Auswaschcn die Rauchbestandteile aus der gehärteten Matrix entfernt, wobei in der Matrixschicht Poren zurückbleiben, in welche die Moleküle der Rauchbestandteile exakt hineinpassen. Eventuell werden die Rauchgasmoleküle in den Poren sogar mit schwachen chemischen Bindungen fixiert.
■ Neben den selektiven Sensoren werden auch nicht-selektive hergestellt, wobei der eine Sensortyp unter der Einwirkung von Rauch bestimmte Eigenschaften stärker ändert als der andere Typ. Der auf Rauch weniger stark reagierende Sensortyp wird als Referenzsensor eingesetzt.

## Patentansprüche

1. Brandmelder mit einem Sensor (6, 7) zur Rauchdetektion und mit einer Auswerteschaltung (5) für die Signale des Sensors, dadurch gekennzeichnet, dass der Sensor aus einem eine Sensorschicht mit molekularer Prägung für den Rauchnachweis aufweisenden Rauchsensor (6) und aus einem Referenzsensor (7) mit einer vom Rauchsensor abweichenden Rauchselektivität besteht.

2. Brandmelder nach Anspruch 1, dadurch gekennzeichnet, dass der Rauch- und der Referenzsensor (6 bzw. 7) je durch einen Massensensor gebildet sind.

3. Brandmelder nach Anspruch 2, dadurch gekennzeichnet, dass der Rauch- und der Referenzsensor (6 bzw. 7) je durch einen SAW-Resonator oder einen Cantilever oder einen Quarz-Dickenschwinger gebildet sind.

4. Brandmelder nach Anspruch 3, dadurch gekennzeichnet, dass der Rauch- und der Referenzsensor (6 bzw. 7) durch Arrays von Cantilevern gebildet sind, und dass verschiedene Cantilever des Rauchsensors (6) eine Selektivität für verschiedene Rauchtypen aufweisen.

5. Verfahren zur Herstellung eines Sensors mit molekularer Prägung zur Rauchdetektion, dadurch gekennzeichnet, dass in ein Gemisch von Matrixmolekülen Rauchgasmoleküle eingebracht werden und die dadurch entstehende Mischung auf ein Substrat aufgebracht und gehärtet wird, und dass anschliessend die Rauchgasmoleküle aus der Matrix entfernt werden, wobei in dieser Poren zurückbleiben, in welche die Rauchgasmoleküle exakt hineinpassen.

6. Verfahren nach Anspruch 5, dadurch gekennzeichnet, dass die genannte Mischung auf das Substrat eines Massensensors aufgebracht wird.

7. Verfahren nach Anspruch 6, dadurch gekennzeichnet, dass die Härtung des Gemisches durch Polymerisierung erfolgt.

8. Verfahren nach Anspruch 5 oder 6, dadurch gekennzeichnet, dass die Rauchgasmoleküle durch Erwärmung oder Auswaschen aus der Matrix entfernt werden.

9. Verfahren nach einem der Ansprüche 5 bis 8, dadurch gekennzeichnet, dass die Rauchgasmoleküle durch Abtrennung aus den Rauchgasen verschiedener Feuer gewonnen werden.

10. Verfahren nach einem der Ansprüche 5 bis 8, dadurch gekennzeichnet, dass die Rauchgasmoleküle aus einer synthetisch hergestellten Mischung von Anteilen der Rauchgase verschiedener Feuer bestehen.
